## Europäisches Patentamt

## European Patent Office

## Office européen des brevets

(11) Publication number: **0 142 514**
**B1**

# EUROPEAN PATENT SPECIFICATION

(45) Date of publication of patent specification: 03.10.90

(51) Int. Cl.⁵: **A 61 M 5/14**

(21) Application number: 84901466.7

(22) Date of filing: 02.03.84

(88) International application number:
PCT/US84/00302

(87) International publication number:
WO 84/03445 13.09.84 Gazette 84/22

(54) INTRAVENOUS DRUG ADMINISTRATION APPARATUS.

(30) Priority: 07.03.83 US 472926

(43) Date of publication of application:
29.05.85 Bulletin 85/22

(45) Publication of the grant of the patent:
03.10.90 Bulletin 90/40

(84) Designated Contracting States:
AT BE CH DE FR GB LI LU NL SE

(56) References cited:
US-A-1 655 664     US-A-4 259 952
US-A-3 254 647     US-A-4 267 837
US-A-3 343 538     US-A-4 424 056
US-A-4 258 723

Journal of Pharmaceutical Sciences, vol. 57, no. 6, June 1968 LOO et al.: "New method for calculating the intrinsic absorption rate of drugs", pages 918-928

American Heart Journal, vol. 102, no. 5, November 1981, STARGEL et al., "Clinical comparison of rapid infusuion and multiple injection methods for lidocaine loading", pages 872-876

(73) Proprietor: THE VANDERBILT UNIVERSITY
Box 506 peabody Campus
Nashville, TN 37203 (US)

(72) Inventor: RIDDELL, James, Gray
42 Malone Hill Park
Belfast BT9-6RE (GB)

(74) Representative: Downey, William Gerrard et al
WILSON, GUNN, ELLIS & CO. 41 Royal Exchange
Cross Street
Manchester M2 7BD (GB)

(56) References cited:
The Journal of Pharmacology and Experimental Therapeutics, Volume 174, No. 1, 1970 BOYES et al. "Oral Absorption and Disposition Kinetics of Lidocaine Hydrochloride in Dogs". Pages 1-8

The Journal of Pharmacology and Experimental Therapeutics. Volume 150, 1965, FLOUIkES, "On the Mechanism of Chlorothiazide-induced Kaliuresis in the Rabbit" Pages 406-407

Courier Press, Leamington Spa, England.

**Description**

Intravenous solutions, such as saline or glucose solutions, are often used as carriers for the continuous administration of drugs to patients at controlled infusion rates. The drugs may prepackaged in intravenous solution bags or bottles. The drug-containing intravenous solution may be administered by gravity flow, using a drip chamber and rate control valve, or a drip chamber with automatic rate controller. Infusion pumps may also be employed which can be preset to provide a constant solution volume delivery rate, and thereby a selected rate of the drug dissolved in the intravenous solution at a preestablished concentration.

A constant rate drug infusion apparatus, as described above, is not a clinically adequate administration means for drugs where a rapid achievement of therapeutic level is needed followed by the maintenance of a highly critical serum level to obtain therapeutic effectiveness over a long period of administration without adverse side effects. Bolus injections of the drug can be used to provide a rapid serum concentration followed by a constant rate infusion, but the transition period between the loading injection and the achievement of a stable serum level at the desired therapeutic concentration is not effectively controlled. A rapid injection followed by a constant slow rate infusion has the disadvantage that there is a considerable time during the early hours when the plasma concentrations are sub-therapeutic. A staged infusion has therefore been proposed and used to some extent, a fast constant infusion being used following the loading injection, and then by a slow constant infusion. Multiple further rapid injections may also be used to augment a slow constant infusion. All of these procedures have the disadvantages that they require attention at strictly defined times if the plasma concentration is not to rise to a toxic level or to a level giving undesired side effects, or, on the other hand, to fall to a sub-therapeutic level.

When a drug is administered intravenously the early plasma drug concentrations are much higher then the drug concentrations in tissues with a poor blood supply. With time the drug distributes to these other tissues and a steady state of distribution is reached. Once this distribution in the body has reached steady state, the aim is to maintain the steady state of infusing drug at the same rate at which is is eliminated from the body.

The problem of developing a simple apparatus for optimum intravenous drug administration has been particularly studied in connection with the administration of anti-arrhythmic drugs such as lidocaine. The effectiveness of lidocaine depends on the rapid achievement and maintenance of rather narrow therapeutic plasma levels, viz. 1.5 to 5.5 micrograms/milliliter. Concentrations above the critical range have been associated with toxic effects including convulsions, coma, and respiratory arrest, while lower concentrations do not adequately protect the patient against a life-threatening arrhythmia. See Salzer, et al, *Clin. Pharmacol. Ther.*, 29 (5) 617—624 (1981); and Stargel, et al, *Amer. Heart J.*, 102 872—876 (1981). It has been proposed that the ideal transitional infusion between a loading injection and a constant rate infusion would be an exponentially decreasing drug infusion rate. This has been approximated by using mechanical constant rate infusion pumps and stepped decreases in the delivery rate. See Vaughn, et al, *Europ. J. Clin. Pharmacol.* 10, 433—440; and Loo, et al, *J. Pharm. Scic.*, 57, 918—928 (1968).

Dilutional methods for achieving an exponentially decreasing delivery rate for lidocaine or other drugs have been tested with experimental animals. Boyes, et al, *J. Pharmacol. Exp. Ther.*, 174, 1—8 (1970) and Foulkes, *J. Pharmacol. Exp. Ther.*, 150, 406—413 (1965). As described in these references an intravenous solution containing no drug is used for progressive dilution of a solution containing the drug. The solutions were mixed by a magnetic or mechanical stirrer. The diluted mixture was delivered to experimental animals. While this procedure is highly desirable from a theoretical standpoint, no simple practical apparatus has heretofore been developed which safely and automatically achieves an exponentially decreasing concentration of the drug.

Summary of invention

The intravenous drug administration apparatus of this invention provides a simple and automatic manner a first adminstration phase in which the drug is infused at an exponentially decreasing concentration from a high loading concentration down to a maintenance concentration, and a second phase in which there is continued delivery of the low maintenance concentration. Observation and resettings are minimized. The patient may be given one or more loading injections, such as for the administration of lidocaine as anti-arrhythmic drug, and then the infusion is started to automatically provide the exponentially decreasing phase during the transitional period while the drug is distributing from the blood into the tissues, and thereafter the maintenance concentration being adequate to replace the drug as it is eliminated from the body.

According to the present invention, there is provided intravenous drug administration apparatus in which a solution containing a drug is progressively diluted prior to delivery, comprising a closed container with a first sterile intravenously administerable aqueous carrier therein, a closed rigid mixing vessel of smaller volume then said container having a second sterile intravenously administerable aqueous carrier providing a high loading concentration of an intravenously administerable drug, said vessel having inlet means connected to an outlet of said container by conduit means, said inlet means providing a solution mixing action within said vessel, said vessel having outlet means positioned and arranged with respect to the inlet means so that mixing of said carriers can occur within said vessel for discharging an admixed carrier to said outlet means, and delivery conduit means connected to the outer end of said outlet means for intravenous delivery of the admixed carrier to a patient, characterised in that said first carrier has said drug therein at a low maintenance concentration, and that the outlet of said container is positionable at the bottom thereof for gravity outflow whereby there can be delivered in a first administration phase an exponentially decreasing concentration of said drug from said high loading concentration down to said maintenance concentration, and in a second phase a continued delivery of said low maintenance concentration.

In use, the inlet is connected to the larger volume low concentration carrier, and the outlet to the infusion cannula connected to the patient. In a preferred arrangement, hypodermic needles are employed to provide the inlets and outlets for the mixing vessel, a short needle extending into the lower portion of the vessel and functioning as the inlet, and a long needle extending into the upper portion of the vessel and providing the outlet.

In a preferred application, the drug in the solutions of the apparatus is lidocaine. However, the apparatus is adaptable for use with many other drugs which are capable of being administered intravenously in aqueous solutions.

Details description

The intravenous drug administration apparatus of this invention utilizes as one of its components, a closed container with a first sterile intravenously administerable aqueous solution therein. This container may be a standard intravenous solution bag or bottle containing 0.5 or 1.0 liters of an intravenous solution, such as normal saline solution, a glucose solution, etc.

The drug to be administered is dissolved in the intravenous solution at a low maintenance concentration. For example, the drug may be lidocaine dissolved in the amount of 2,000 mg in 1,000 ml of normal saline, giving a concentration of 2 mg/ml. Other anti-arrhythmic drugs such as mexiletine can be administered, as well as drugs for other conditions. For example, the apparatus may be used for the administration of an anaesthetic agent such as methohexitone, a respiratory stimulant such as doxapram, a drug for premature labor such as ritodrine or salbutamol, a drug for acute asthmatic attacks such as theophylline, etc. The large volume container providing a solution of the drug at the low maintenance concentration will have an outlet positionable at the bottom thereof for gravity outflow, as is well known with respect to intravenous solution bags and bottles. A container may be provided with an inlet port for introduction of the drug, or the drug may be prepackaged in the container.

The apparatus also includes a closed rigid mixing vessel of smaller volume having a second sterile intravenously administerable aqueous solution providing a high loading concentration of the drug. Typically, the intravenous solution will be the same as that in the larger volume container, that is, it will be a saline solution, a glucose solution, etc., and the same drug will be dissolved in the second solution but at a considerably higher concentration, the concentration corresponding to a high loading concentration of the drug, as desired for an initial infusion of the patient. For example, where the drug is lidocaine and a normal saline solution is used, the mixing vessel and the solution therein may have a volume of 20 ml and may contain 200 mg lidocaine, giving a concentration of 10 mg/ml. It will be understood that the relative concentrations and relative volumes of the first and second solutions are subject to wide variations, depending on the particular drug being administered, and the desired length of the period of administration. However, in general, the loading concentration in the second solution will be at least three times that of the maintenance concentration in the first solution, and the volume of the first solution will be at least five times that of the second solution.

The mixing vessel is provided with an inlet connected to the container outlet by conduit means, and the inlet means is designed to provide a solution mixing action within the vessel. The vessel is also provided with outlet means positioned and arranged with respect to the inlet means so that mixing of the solutions can occur within the vessel for discharging an admixed solution to the outlet. A delivery conduit is connected to the outlet for intravenous delivery of the admixed progressively diluted solution to a patient during a first phase of the administration. In the second phase, with the continued administration of the maintenance solution no mixing dilution is needed. The concentration of the drug in the solution of the mixing vessel is at the same level as that of the larger volume container.

The apparatus can be operated entirely by gravity flow. The container with the low concentration drug is positioned at a higher level than the mixing vessel, and the solution is permitted to flow therefrom and into the mixing vessel under the action of gravity. However, it may be preferred to employ a constant rate pump. The pump may be preset to a desired flow rate, and interposed between the container and the mixing vessel. The low concentration solution is delivered from the outlet side of the pump to the mixing vessel. Several suitable pumps are commercially available, such as the Abbott/Shaw Life Care Pump. (See also U.S. Patent 3,620,650).

Other standard components of intravenous administration sets will preferably be included. For example, a drip chamber and a rate control clamp, such as roller clamp, may be interposed between the low concentration container and the mixing vessel.

Other commonly employed devices can be used, such as automatic rate controller to assure the maintenance of a constant drip rate. There will also usually be provided a shut off clamp on the downstream conduit between the mixing vessel and the cannula attached to the patient.

The intravenous solution bag, which contains the low concentration solution of the drug, is provided with a hanger for mounting it at a level above the mixing vessel to provide for gravity flow between the solution bag and the mixing vessel. In the illustration given, the bag has a lower outlet tube connecting to a drip chamber, which in turn is connected to the inlet of the mixing vessel by a conduit, which is provided with a roller clamp to provide for the drip rate adjustment. The lower end of the solution bag is also provided with an access port tube through which a drug may be introduced into the intravenous solution within the bag. Preferably, however, the drug is prepackaged, in which case an access port will not be needed. A conventional drip chamber is provided below the intravenous solution bag, and connected by a conduit to the mixing vessel. A roller clamp may be mounted on the conduit adjacent the drip chamber for adjusting the drip rate, and thereby predetermining the constant flow rate. Alternatively, as described above, an automatic controller can be used. Further, a constant rate pump may be interposed in the conduit for passing the solution into the mixing vessel at a constant preset rate.

The mixing vessel will contain the sterile aqueous solution of the drug at a high concentration corresponding to the desired loading concentration. In the example given, the mixing vessel consists of a small bottle or vial, having a rubber closure plug through which extend two hollow needles, a short inlet needle and a long outlet needle. The plug with the needles frictionally held therein is secured in the neck of the vial by means of a metal clamping ring. The needles may comprise commercially available standard hypodermic needles, or special mixing needles may be designed for this application. The inlet needle terminates in the lower portion of the mixing vessel, while the outlet needle extends into and receives solution from the upper portion of the vessel. With this arrangement, mixing action occurs within the vessel. The solution introduced through the inlet needle enters as a fine stream jet, which promotes mixing and turbulence within the vessel. Since the ends of the inlet and outlet needles are separated, mixing can occur within the vessel, so that an admixed solution is discharged through the outlet needle. The outer end of the outlet needle is connected to a conduit for delivery to the venous cannula of the patient. As shown, a slide clamp is mounted on the outlet conduit, which may be moved from an open position to a clamping position cutting off the flow.

With the above described apparatus, after the patient has been given one or more injections of the drug to produce immediate loading, the slide clamp may be opened to permit the beginning of the infusion. At the start of the infusion, the drug will be administered at the high concentration of the mixing vessel. As the administration proceeds, the low concentration solution from the bag will progressively mix with and dilute the solution in the mixing vessel, thereby providing a close approximation to an exponentially reducing cencentration as delivered to the patient. Since the volume of solution in the bag is much greater than that in the mixing vessel, a condition will be reached at which there is remaining solution in the bag, while the concentration of the solution in the mixing vessel is the same as that of the bag. Thereafter, the continued administration will be at the low maintenance concentration of the bag solution. During all phases of the administration, there will be a minimal need to monitor or attend the administration apparatus. The transitional infusion with exponentially reducing concentration and the continued infusion thereafter at the maintenance rate wil be achieved automatically.

In an illustrative embodiment, a one liter intravenous solution bag containing 0.9% saline can be used. 10 ml lidocaine HCL (200 mg/ml) is introduced into the bag by an additive syringe, resulting in a lidocaine HCL concentration of 2 mg/ml (1.7 mg/ml lidocaine). The mixing vessel can consist of a 20 ml vial of prepackaged lidocaine solution containing 200 mg lidocaine HCL (8.7 mg/ml lidocaine). The vial may have a rubber closure through which hypodermic needles are inserted. The vial closure is pierced with two needles, the needle providing the inlet being $\frac{1}{2}'' \times 27$ SWG hypodermic needle, and the outlet being provided by a $1\frac{1}{2}'' \times 22$ SWG hypodermic needle. The inlet needle terminates in the lower portion of the inverted container, and the outlet needle extended into the upper portion. Preferably, although not essentially, the mixing vessel is completely full of solution, thereby eliminating any air space which might create air bubbles in the infusion solution.

In a delivery rate experiment, a constant rate pump was interposed in the conduit between the solution bag and the mixing vessel. The dye indocyanine green was substituted for the lidocaine at the same concentrations. The pump was set to deliver solution at a rate of 1 ml/min. As the solution jetted into the mixing vessel through the inlet needle, a mixing action was created, and mixed solution was continuously removed at the same rate through the outlet needle. The dye concentration as determined spectrophotometrically fell from 10 mg/ml to the concentration of 2 mg/ml by a close approximation to a predicted exponential reduction curve. After 15 minutes the concentration of the delivered solution had fallen to 5.6 mg/ml (predicted 5.8), after 30 minutes to 2.3 mg/ml (predicted 2.4) after 90 minutes to 2.0 mg/ml (predicted 2.1), and after 120 minutes continued at 2.0 mg/ml (predicted 2.0 mg/ml).

In use of the apparatus, total delivery time will depend on the volume of the low concentration solution. For example, with a 1 liter volume and a flow rate of 1 ml/min infusion can run for approximately 16.7 hours. At the same rate with a low concentration solution volume of 0.5 liters, the infusion can be continued for approximately 8.3 hours.

In commercial embodiments of the apparatus of this invention, it will be preferred to have the drug prepackaged in both the intravenous solution bag and in the mixing vessel. Further, the mixing vessel may be provided in the form of a vial equipped with a previously described intlet and outlet hypodermic needle. Closure caps may be provided for the projecting ends of the needles, which can be removed to permit their attachments to the appropriate conduits. Both the bag and the mixing vessel may be supplied as components of a complete intravenous administration set.

## Claims

1. Intravenous drug administration apparatus in which a solution containing a drug is progressively diluted prior to delivery, comprising a closed container with a first sterile intravenously administerable aqueous carrier therein, a closed rigid mixing vessel of smaller volume than said container having a second sterile intravenously administerable aqueous carrier providing a high loading concentration of an intravenously administerable drug, said vessel having inlet means connected to an outlet of said container by conduit means, said inlet means providing a solution mixing action within said vessel, said vessel having outlet means positioned and arranged with respect to the inlet means so that mixing of said carriers can occur within said vessel for discharging an admixed carrier to said outlet means, and delivery conduit means connected to the outer end of said outlet means for intravenous delivery of the admixed carrier to a patient, characterised in that said first carrier has said drug therein at a low maintenance concentration, and that the outlet of said container is positionable at the bottom thereof for gravity outflow whereby there can be delivered in a first administration phase an exponentially decreasing concentration of said drug from said high loading concentration down to said maintenance concentration, and in a second phase a continued delivery of said low maintenance concentration.

2. The apparatus of claim 1 in which said drug is lidocaine.

3. The apparatus of claim 1 in which said drug is lidocaine and said carriers are normal saline solutions.

4. The apparatus of claim 1 in which said first carrier is supplied to said mixing vessel entirely by gravity flow.

5. The apparatus of claim 1 in which said inlet means comprises a hollow needle terminating in the lower portion of said vessel and said outlet means comprises a hollow needle having its inlet end located in the upper portion of said vessel.

6. The apparatus of claim 1 in which said loading concentration is at least three times that of said maintenance concentration, and said first carrier has a volume at least five times that of said second carrier.

## Patentansprüche

1. Vorrichtung zur intravenösen Verabreichung von Medikamenten unter progressiver Verdünnung einer ein Mdikament enthaltenden Lösung vor der Abgabe, mit einem geschlossenen Behälter, der einen ersten, sterilen, intravenös verabreichbaren, wässerigen Träger enthält, ferner mit einem geschlossenen, starren Mischgefäss von kleinerem Volumen als der genannte Bahälter, das einen zweiten, sterilen, intravenös verabreichbaren wässerigen Träger mit einer hohen Anfangskonzentration eines intravenös verabreichbaren Medikaments enthält, wobei das Mischgefäss Einlassmittel aufweist, die über Lietungsmittel mit einem Auslass des genannten Behälters verbunden sind und im Mischgefäss einen Lösungsmischvorgang bewirken, wobei ferner das Mischgefäss Auslassmittel aufweist, die bezüglich den Einlassmitteln in der Weise angeordnet sind, dass im Mischgefäss eine Vermischung der genannten Träger zur Abgabe eines zugemischten Trägers an die genannten Auslassmittel erfolgt, und wobei ferner das äussere Ende der genannten Auslassmittel mit Infusionsleitungen zur intravenösen Verabreichung des gemischten Trägers an einen Patienten verbunden ist, dadurch gekennzeichnet, dass der erste Träger das genannte Medikament in einer schwachen Erhaltungskonzentration enthält, und dass der Auslass des Behälters zwecks Schwerkraftabfluss an dessen Unterseite befestigbar ist, wobei in einer ersten Verabreichungsphase eine von der genannten hohen Aufangskonzentration zur genannten schwachen Erhaltungskonzentration exponentiell abnehmende Konzentration des Mediakaments verabreichbar ist, und in einer zweiten Phase eine kontinuierliche Verabreichung der genannten schwachen Erhaltungskonzentration erfolgen kann.

2. Vorrichtung nach Anspruch 1, dadurch gekennzeichnet, dass das Medikament Lidocain ist.

3. Vorrichtung nach Anspruch 1, dadurch gekennzeichnet, dass das Medikament Lidocain und die Träger normale Salzlösungen sind.

4. Vorrichtung nach Anspruch 1, dadurch gekennzeichnet, dass die Zuleitung des ersten Trägers zum Mischgefäss ausschliesslich durch Gravitätsfluss erfolgt.

5. Vorrichtung nach Anspruch 1, dadurch gekennzeichnet, dass die Einlassmittel eine Hohlnadel aufweisen, welche im unteren Bereich des Mischgefässes endet, und die Auslassmittel eine Hohlnadel aufweisen, deren Einlass im oberen Teil des Mischgefässes angeordnet ist.

6. Vorrichtung nach Anspruch 1, dadurch gekennzeichnet, dass die genannte Anfangskonzentration mindestens drei mal höher ist als die Erhaltungskonzentration, und das Volumen des ersten Trägers mindestes das Füffache des Volumens des zweiten Trägers beträgt.

## Revendications

1. Appareil d'administration intraveineuse d'un médicament, dans lequel une solution contenant un médicament est progressivement diluée avant sa sortie, comprenant un récipient fermé, à l'intérieur duquel se trouve un premier support stérile aqueux administrable par voie intraveineuse, un réservoir de mélange rigide, fermé, de plus petit volume que ledit récipient, ayant un second support stérile aqueux administrable par voie intraveineuse, fournissant une concentration de charge élévée d'un médicament administrable par voie intraveineuse, ledit réservoir ayant un moyen d'entrée connecté à une sorte dudit récipient par un conduit, ledit moyen d'entrée assurant une action de mélange de la solution dans ledit réservoir, ledit réservoir ayant un moyen de sortie positionné et agencé par rapport au moyen d'entrée de manière que le mélange desdits supports puisse s'opérer dans ledit réservoir pour décharger un support mélangé audit moyen de sortie, et un conduit de décharge connecté à l'extrémité de sortie dudit moyen de sortie pour fourniture intraveineuse de support mélangé à un malade, caractérisé par le fait que ledit premier support contient ledit médicament à une faible concentration d'entretien et que la sortie dudit récipient peut être positionnée au fond de ce dernier pour écoulement par gravité, grâce à quoi peut être fournie, dans une première phase d'administration, une concentration dudit médicament décroissant exponentiellement de ladite haute concentration de charge à ladite concentration d'entretien et, dans une seconde phase, une fourniture continue de ladite faible concentration d'entretien.

2. Appareil selon la revendication 1, dans lequel ledit médicament est la lidocaïne.

3. Appareil selon la revendication 1, dans lequel ledit médicament est la lidocaïne et lesdits supports sont des solutions salines normales.

4. Appareil selon la revendication 1, dans lequel ledit premier support est fourni au dit réservoir de mélange entièrement par écoulement par gravité.

5. Appareil selon la revendication 1, dans lequel ledit moyen d'entrée comprend une aiguille creuse se terminant à la portion inférieure dudit réservoir et ledit moyen de sortie comprend une aiguille creuse ayant son extrémité d'entrée dans la partie supérieure dudit réservoir.

6. Appareil selon la revendication 1, dans lequel ladite concentration de charge est au moins triple de ladite concentration d'entretien et ledit premier support a un volume au moins quintuple de celui dudit second support.